# FASCICULE DE BREVET EUROPEEN

(11) **EP 0 759 469 B1**
(45) Date de publication et mention de la délivrance du brevet: **19.03.2003**
(21) Numéro de dépôt: 95810497.8
(22) Date de dépôt: 07.08.1995
(51) Int. Cl.: C12N 15/31, C07K 14/305, C12N 1/20, A23B 4/22, A61K 7/16, A61K 7/02

(54) **Bactériocine**
Bakteriozin
Bacteriocin

(43) Date de publication de la demande: 26.02.1997
(73) Titulaire: SOCIETE DES PRODUITS NESTLE S.A., 1800 Vevey (CH)
(72) Inventeur: Mollet, Beat, 1074 Mollie-Margot (CH); Peel, John, CH-1470 Lully (CH); Pridmore, David, 1000 Lausanne 26 (CH); REKHIF, Nadji, CH-1010 Lausanne (CH); Suri, Bruno, CH-4416 Bubendorf (CH)
(74) Mandataire: Wavre, Claude-Alain

(56) Documents cités:
- IND. ALIMENT. (PINEROLO, ITALY) (1992), 31(306), 660-5 CODEN: INALBB;ISSN: 0019-901X, 1992 CANTONI, C. ET AL 'Bacteriocins of Micrococcaceae'
- JOURNAL OF BIOLOGICAL CHEMISTRY, vol. 268, no. 22, 5 Août 1993 MD US, pages 16361-16368, JEAN-CHRISTOPH PIARD ET AL. 'Structure, organization, and expression of the lct gene for Lacticin 481, a novel Lantibiotic produced by Lactococcus lactis'

### LISTE DE SEQUENCES

(1) INFORMATIONS GENERALES:
   (i) DEPOSANT:
      (A) NOM: SOCIETE DES PRODUITS NESTLE
      (B) RUE: AVENUE NESTLE 55
      (C) VILLE: VEVEY
      (D) ETAT OU PROVINCE: CANTON DE VAUD
      (E) PAYS: SWITZERLAND
      (F) CODE POSTAL: 1800
      (G) TELEPHONE: (041) 21 924 34 20
      (H) TELECOPIE: (041) 21 924 28 80
   (ii) TITRE DE L' INVENTION: BACTERIOCINE DE MICROCOCCUS VARIANS
   (iii) NOMBRE DE SEQUENCES: 8
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 25 acides aminés
      (B) TYPE: acide aminé
      (C) NOMBRE DE BRINS:
      (D) CONFIGURATION: linéaire
   (ii) TYPE DE MOLECULE: protéine
   (vi) ORIGINE:
      (A) ORGANISME: MICROCOCCUS VARIANS
      (C) INDIVIDU/ISOLE: DEUX CLONES CNCM I-1586 et CNCM I-1587
   (xi) DESCRIPTION DE LA SEQUENCE: SEQ ID NO: 1:
   Gly Ser Gly Val Ile Pro Thr Ile Ser His Glu Cys His Met Asn Ser
   1               5                   10                  15
   Phe Gln Phe Val Phe Thr Cys Cys Ser
               20                  25
(2) INFORMATIONS POUR LA SEQ ID NO: 2:
   (i) CARACTERISTIQUES DE LA SEQUENCE:
      (A) LONGUEUR: 278 paires de bases
      (B) TYPE: nucléotide
      (C) NOMBRE DE BRINS: double
      (D) CONFIGURATION: linéaire
   (ii) TYPE DE MOLECULE: ADN (génomique)
   (vi) ORIGINE:
      (A) ORGANISME: MICROCOCCUS VARIANS
      (C) INDIVIDU/ISOLE: DEUX CLONES CNCM I-1586 et CNCM I-1587
   (ix) CARACTERISTIQUE:
      (A) NOM/CLE: CDS
      (B) EMPLACEMENT:88..228
   (ix) CARACTERISTIQUE:
      (A) NOM/CLE: sig_peptide
      (B) EMPLACEMENT:88..153
   (ix) CARACTERISTIQUE:
      (A) NOM/CLE: mat-peptide
      (B) EMPLACEMENT:154..228
   (xi) DESCRIPTION DE LA SEQUENCE: SEQ ID NO: 2:
   GTTGAAAATC ATCCGGAAGG ATGATTCTGT GTTCATGCGA GGCCACCCGG AATCGCGGCA      60
   GCCACACCCA CTGAGAGGAC TAGAACA ATG ACG AAC GCA TTT CAG GCA CTG         111
                                 Met Thr Asn Ala Phe Gln Ala Leu
                                 -22     -20                 -15
   GAC GAA GTC ACG GAC GCC GAG CTC GAC GCC ATC CTT GGC GGG GGC AGT      159
   Asp Glu Val Thr Asp Ala Glu Leu Asp Ala Ile Leu Gly Gly Gly Ser
                   -10                 -5                  1
   GGT GTT ATT CCC ACG ATC AGC CAC GAG TGC CAC ATG AAC TCC TTC CAG      207
   Gly Val Ile Pro Thr Ile Ser His Glu Cys His Met Asn Ser Phe Gln
               5                   10                  15
   TTC GTG TTC ACC TGC TGC TCC TGAGAAACTC CTCCGATGCT CAGAGGGCCG          258
   Phe Val Phe Thr Cys Cys Ser
        20                  25
   CGCTAGGAAA ATCTAGTAAG                                                 278
(2) INFORMATIONS POUR LA SEQ ID NO: 3:
   (i) CARACTERISTIQUES DE LA SEQUENCE:
      (A) LONGUEUR: 47 acides aminés
      (B) TYPE: acide aminé
      (D) CONFIGURATION: linéaire
   (ii) TYPE DE MOLECULE: protéine
   (xi) DESCRIPTION DE LA SEQUENCE: SEQ ID NO: 3:
   Met Thr Asn Ala Phe Gln Ala Leu Asp Glu Val Thr Asp Ala Glu Leu
   -22     -20                 -15                 -10
   Asp Ala Ile Leu Gly Gly Gly Ser Gly Val Ile Pro Thr Ile Ser His
       -5                  1               5                   10
   Glu Cys His Met Asn Ser Phe Gln Phe Val Phe Thr Cys Cys Ser
                   15                  20                  25
(2) INFORMATIONS POUR LA SEQ ID NO: 4:
   (i) CARACTERISTIQUES DE LA SEQUENCE:
      (A) LONGUEUR: 23 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (iii) HYPOTHETIQUE: OUI
   (xi) DESCRIPTION DE LA SEQUENCE: SEQ ID NO: 4:
   TGGCARTTYG TNTTYACNTG YTG 23
(2) INFORMATIONS POUR LA SEQ ID NO: 5:
   (i) CARACTERISTIQUES DE LA SEQUENCE:
      (A) LONGUEUR: 44 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 5:
   GGNTCNGGNG TNATHCAYAC NATHTCNCAY GARTGYAAYA TGAA 44
(2) INFORMATIONS POUR LA SEQ ID NO: 6:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 6:
   ATAACAATTT CACACAGG 18
(2) INFORMATIONS POUR LA SEQ ID NO: 7:
   (i) CARACTERISTIQUES DE LA SEQUENCE:
      (A) LONGUEUR: 18 paires de bases
      (B) TYPE: nucléotide
      (C) NOMBRE DE BRINS: simple
      (D) CONFIGURATION: linéaire
   (ii) TYPE DE MOLECULE: Autre acide nucléique
      (A) DESCRIPTION: /desc = "oligonucleotide"
   (xi) DESCRIPTION DE LA SEQUENCE: SEQ ID NO: 7:
   GGTTTTCCCA GTCACGAC                                                   18
(2) INFORMATIONS POUR LA SEQ ID NO: 8:
   (i) CARACTERISTIQUES DE LA SEQUENCE:
      (A) LONGUEUR: 7 acides aminés
      (B) TYPE: acide aminé
      (C) NOMBRE DE BRINS: simple
      (D) CONFIGURATION: linéaire
   (ii) TYPE DE MOLECULE: peptide
   (xi) DESCRIPTION DE LA SEQUENCE: SEQ ID NO: 8:
   Lys Gly Gly Ser Gly Val Ile
   1               5

## Description

La présente invention a pour objet une bactériocine, une souche productrice de cette bactériocine, un procédé de préparation de cette bactériocine et l'utilisation de cette bactériocine et/ou d'une souche productrice de cette bactériocine dans la fabrication de produits alimentaires et de produits cosmétiques.

### ETAT DE LA TECHNIQUE

Des bactériocines ont été isolées chez de nombreuses bactéries, Gram positives et Gram négatives. Ce sont des molécules de nature essentiellement protéique ayant un pouvoir bactériocide et, de ce fait, provoquant une réaction antagoniste entre la bactérie qui la produit et une ou plusieurs autres espèces bactériennes. En outre, le spectre d'inhibition des bactériocines est souvent limité aux espèces proches de l'espèce de la bactérie qui la produit.

On a mis en évidence notamment des bactériocines chez les bactéries lactiques. Par exemple, EP 0643136 (Société des produits Nestlé) décrit l'identification de deux bactériocines de *Streptococcus thermophilus*.
De même, une bactériocine a été isolée de *Lactococcus lactis* (App. and Env. Microbio. 58, 279-284, 1992 ; J. of Bio. Chem. 268, 16361-16368, 1993).

Mais, à ce jour, on ne connaît aucune bactériocine de *Micrococcus varians*. Or, *Micrococcus varians* est très utilisée dans le domaine alimentaire, notamment dans la fermentation de la viande pour la fabrication de produits de charcuterie, tels que les salamis et les saucisses, par exemple. Il serait donc très utile de disposer d'une souche productrice d'une bactériocine, de manière à lutter contre des agents pathogènes.

La présente invention a pour but de répondre à ce besoin.

### RESUME DE L'INVENTION

A cet effet, la bactériocine selon la présente invention est une bactériocine de *Micrococcus varians* présentant la séquence en acides aminés SEQ ID NO:1 ou toute séquence en acides aminés différant de la séquence SEQ ID NO:1 par une substitution, une délétion et/ou une insertion de 1 à 4 acides aminés.

De plus, tout fragment nucléotidique codant pour cette bactériocine, notamment le fragment nucléotidique présentant la séquence SEQ ID NO:2, entre également dans le cadre de la présente invention.

De même, la souche selon la présente invention est une souche de *Micrococcus varians* producrice de cette bactériocine, notamment les souches de *Micrococcus varians* CNCM I-1586 et CNCM I-1587.

Dans le procédé de préparation de la bactériocine selon la présente invention, on cultive, en milieu et en conditions favorables de croissance, une souche de *Micrococcus varians* productrice de la bactériocine, notamment la souche CNCM I-1586 ou la souche CNCM I-1587, de manière à obtenir un milieu de culture contenant 10⁷ à 10¹¹ germes de cette souche par ml, on centrifuge la culture obtenue, puis on prépare un extrait du surnageant contenant la bactériocine.

Enfin, l'utilisation de la bactériocine de *Micrococcus varians* selon l'invention comprend l'utilisation de sa séquence nucléotidique, ainsi que de sa séquence signale, et l'utilisation d'extrait de surnageant contenant la bactériocine et d'une souche de *Micrococcus varians* productrice de la bactériocine pour la préparation de produits alimentaires et de produits cosmétiques.

### DESCRIPTION DETAILLEE DE L'INVENTION

Dans la suite de l'exposé, la bactériocine selon la présente invention sera dénommée "variacine".

Au sens de la présente invention, une unité arbitraire (ua) est définie comme l'inverse du taux de la plus grande dilution à laquelle un échantillon montre encore un pouvoir bactériocide dans le test connu par l'homme du métier sous la désignation d' "agar well test", expression anglaise qui signifie littéralement test du puits creusé dans l'agar.

Au sens de la présente invention, le terme "fragment" ou "fragment d'ADN" doit être compris comme un fragment d'ADN simple ou double brin codant partiellement ou totalement, et pouvant être synthétisé, reproduit *in vitro* par exemple par la méthode connue appelée "Polymerase chain Reaction", ou reproduit *in vivo* dans une bactérie de type *Escherichia coli*, par exemple.

Au sens de la présente invention, on entend par "fragment homologue" tout fragment ne différant des fragments selon l'invention que par la substitution, la délétion ou l'insertion d'un faible nombre de bases. Dans ce cadre, on considèrera en particulier comme homologue deux fragments d'ADN qui, du fait de la dégénérescence du code génétique, codent pour un même polypeptide. On considèrera aussi comme fragment homologue, celui qui présente plus de 80 % d'homologie avec le fragment selon l'invention. Dans ce dernier cas, l'homologie est déterminée par le rapport entre le nombre de bases d'un fragment homologue et celui d'un fragment selon l'invention.

Enfin, au sens de la présente invention, on entend par "fragment qui s'hybride" tout fragment capable de s'hybrider aux fragments selon la présente invention par la méthode de Southern-Blot (Sambrook et all., Molecular Cloning, A Laboratory Manual, Cold Spring Harbor Laboratory Press, U.S.A, 1989, chapitre 9.31 à 9.58). De préférence, l'hybridation est conduite dans des conditions rigoureuses ou stringentes, de manière à éviter des hybridations aspécifiques ou peu stables.

On a isolé des souches CNCM I-1586 et CNCM I-1587 un facteur protéique, en l'occurrence une bactériocine, ayant un pouvoir bactériocide puissant. On a appelé variacine cette bactériocine de *Micrococcus varians* qui présente donc la séquence en acides aminés SEQ ID NO:1 décrite dans la liste des séquences ci-après.

Vu l'intérêt présenté par la variacine, l'invention concerne également toute bactériocine ayant une séquence en acides aminés différant de la séquence SEQ ID NO:1 par une substitution, une délétion et/ou une insertion de 1 à 4 acides aminés. En effet, ladite bactériocine, présentant une séquence en acides aminés qui diffère de la séquence SEQ ID NO:1 par une substitution, une délétion et/ou une insertion de 1 à 4 acides aminés, peut avoir un spectre d'inhibition à un genre ou une espèce bactérienne plus large que celui de ladite variacine, par exemple.

On a pu aussi sélectionner des deux souches CNCM I-1586 et CNCM I-1587 un fragment nucléotidique chromosomique codant pour la variacine selon l'invention. Ledit fragment présente la séquence SEQ ID NO:2 donné dans la liste des séquences ci-après.

Vu l'intérêt présenté par la présente invention, l'invention concerne aussi tout fragment nucléotidique codant pour la variacine selon la présente invention, notamment les fragments nucléotidiques homologues ou s'hybridant avec la séquence SEQ ID NO:2.

En particulier, l'invention concerne les nucléotides 88 à 153 de la séquence SEQ ID NO:2 codant pour le peptide signal de la variacine, les nucléotides 154 à 228 de la séquence SEQ ID NO:2 codant pour la variacine excrétée selon la présente invention et/ou les nucléotides 88 à 228 de la séquence SEQ ID NO:2 codant pour la bactériocine fusionnée à son peptide signal.

Le fragment codant pour la variacine excrétée peut être utilisé avantageusement pour exprimer la variacine selon la présente invention, dans une plante ou dans un microorganisme autre que *Micrococcus varians*. A cet effet on peut cloner les nucléotides 154 à 228 de la séquence SEQ ID NO:2 dans un vecteur d'expression en aval d'un promoteur et d'une séquence signale, et en amont d'un terminateur, tout en respectant le cadre de lecture, puis on peut introduire ledit vecteur dans une plante, une bactérie ou une levure, de manière à augmenter leur spectre d'inhibition à certaines bactéries, par exemple.

On peut utiliser la séquence signale de l'invention en fusionnant les nucléotides 88 à 153 de la séquence SEQ ID NO:2 à un gène d'intérêt, tout en respectant le cadre de lecture, puis en clonant le tout dans un vecteur d'expression de *Micrococcus varians*, de manière à permettre l'expression et l'excrétion de la protéine codée par ledit gène d'intérêt dans *Micrococcus varians*, par exemple.

On peut cloner les nucléotides 88 à 228 de la séquence SEQ ID NO:2 dans un vecteur d'expression de *Micrococcus varians* et l'introduire dans une autre souche de *Micrococcus varians*, pour que cette dernière produise la variacine selon la présente invention.

De plus, la souche de *Micrococcus varians* comprenant, intégré dans son génome ou par le moyen d'un vecteur d'expression, un fragment d'ADN codant pour la variacine selon l'invention, est également un objet de la présente invention. Notamment les souches de *Micrococcus varians*, déposées le 7 juin 1995, selon le Traité de Budapest, à la Collection National de Cultures de Microorganismes, INSTITUT PASTEUR, 25 Rue du Docteur Roux, F-75724 PARIS CEDEX 15, France, où elles ont reçu les numéros de dépôt CNCM I-1586 et CNCM I-1587, font l'objet de la présente invention.

Les *Micrococcus varians* sont des bactéries Gram positives, catalase positive, aérobes qui sont immobiles de manière permanente. Elles ont une forme sphérique et se présentent sous forme de tétrades arrangées irrégulièrement.
Les colonies de *Micrococcus varians* sont de couleur jaune sur milieu BHI. La température optimale de croissance desdites souches est de 25-37° C.
Les souches CNCM I-1586 et CNCM I-1587, faisant l'objet de la présente invention, métabolisent toutes deux le glucose et le fructose. La souche CNCM I-1587 métabolise en plus le saccharose et le furanose.
De plus, la souche CNCM I-1586 renferme deux plasmides de 4 et 12 kb, alors que la souche CNCM I-1587 n'en renferme qu'un seul de 7 kb.

Les surnageants de culture des souches CNCM I-1586 et CNCM I-1587 présentent un spectre d'inhibition à la croissance d'autres bactéries relativement large. Parmi les bactéries sensibles auxdits surnageants, on peut comprendre *Lactococcus lactis*, *Lactobacillus helveticus, Lactobacillus delbrueckii subsp. bulgaricus, Lactobacillus delbrueckii subsp. lactis, Lactobacillus delbrueckii subsp. delbrueckii, Lactobacillus acidophilus, Lactobacillus johnsonii, Lactobacillus plantarum, Lactobacillus sake, Lactobacillus curvatus, Leuconostoc carnosum, Leuconostoc mesenteroides subsp. mesenteroides, Streptococcus thermophilus, Listeria monocytogenes, Enterococcus faecalis subsp. faecalis, les spores et cellules végétatives de Bacillus subtilis, Bacillus cereus, Bacillus polymyxa, Bacillus circulans, Bacillus pumulus et Bacillus liqueniformis* et les *Clostridia*, par exemple.

De préférence, lors du procédé de préparation de la variacine, on cultive, en milieu et en conditions favorables de croissance, une souche de *Micrococcus varians* productrice de ladite bactériocine, de manière à obtenir un milieu de culture contenant 10⁷ à 10¹¹ germes de ladite souche par ml, on centrifuge la culture obtenue, puis on prépare un extrait du surnageant contenant ladite bactériocine.

Pour produire cet extrait, la souche de *Micrococcus varians* productrice de la variacine, selon la présente invention, notamment la souche CNCM I-1586 ou la souche CNCM I-1587, peut être cultivée dans un milieu et dans des conditions favorables à la croissance de *Micrococcus varians*. A cet effet, on peut notamment la cultiver dans le milieu BHI, à 25-37° C en aérobiose, sous agitation, jusqu'à obtenir une concentration de 10⁷ - 10¹¹ germes par ml de milieu, par exemple. On centrifuge la culture standard, ainsi obtenue, à 4000-6000 g et l'on recueille l'extrait de surnageant contenant ladite bactériocine.

La présente invention concerne également l'utilisation de la variacine, notamment sous forme d'extrait, ou l'utilisation d'une souche de *Micrococcus varians* productrice de cette bactériocine pour la préparation de produits alimentaires et de produits cosmétiques.

On peut utiliser en particulier en culture l'une desdites souches de *Micrococcus varians* selon la présente invention dans la fermentation de la viande pour la préparation de salami, de manière à lutter contre la contamination par *Clostridia*, par exemple.

On peut utiliser la variacine sous forme d'extrait brut ou purifié, ajouté au levain obtenu à partir de bactéries résistantes à ladite variacine dans la préparation de fromages, notamment de fromages du type mozzarella, pour éviter les trous produits par *Bacillus polymixa* dont les spores survivent à la fermentation, et du type vacherin, pour lutter contre la contamination par *Listeria monocytogenes*, par exemple.

On peut utiliser la variacine, notamment sous forme d'extrait brut ou purifié, ou une des deux souches comme additif ou agent actif contre des bactéries pathogènes dans la préparation de mousses dessert tels que les flans pasteurisés, de manière à lutter contre la croissance de spores telles que *Clostridia, Bacillus cereus* ou de souches bactériennes telles que *Listeria*, par exemple.

De plus, on peut utiliser la variacine sous forme d'extrait brut ou purifié, ou une des deux souches comme additif ou agent actif contre des bactéries pathogènes lors de la préparation de produits cosmétiques, comme les crèmes réhydratantes, ou les déodorants, de manière à lutter contre les bactéries pathogènes de la peau, par exemple.

La variacine selon la présente invention est caractérisée plus en détails ci-après à l'aide de différentes données microbiologiques, biochimiques et génétiques illustrant ses propriétés. Les pourcentages sont données en poids.

### Unité d'activité antibactérienne - "Agar Well-Test"

Dans le cadre du présent exposé, on définit le pouvoir bactériocide en termes d'unités arbitraires.

Un surnageant d'une culture classique de *Micrococcus varians* selon la présente invention, préparé par exemple dans les conditions illustrées à l'Exemple 1, présente typiquement une activité de 640 ua/ml. De même, un concentrat dudit surnageant préparé, par exemple dans les conditions illustrées à l'Exemple 2, présente typiquement une activité d'environ 24000 ua/ml.

C'est à l'aide dudit Agar Well Test que l'on détermine si un échantillon montre encore un pouvoir bactériocide à un taux de dilution donné.

Pour ce faire, dans une boîte de Petri, on inocule 15 ml de milieu MRS agar contenant une souche indicatrice à un taux de 10⁵-10⁶ UFC/ml. On utilise comme souche indicatrice, une souche sensible à la variacine, en l'occurrence la souche *Lactobacillus bulgaricus* (YL 5) ou la souche *Lactobacillus helveticus* (N2), par exemple.

On perce des trous de 5 mm de diamètre dans le milieu de culture. On déverse les échantillons du surnageant ou du concentrat du surnageant à tester dans les trous, à raison de 50 µl par trou. On pré-incube durant 2 h à 4° C puis on incube à 30° C ou 37° C, pendant une nuit, selon la souche indicatrice utilisée. Suite à l'incubation, la souche indicatrice a poussé et des halos d'inhibition sont visibles. Le taux de dilution auquel un échantillon ne présente plus de pouvoir bactériocide est le taux de dilution à partir duquel on ne distingue plus de halo d'inhibition.

### Inactivation par des enzymes

A l'aide dudit Agar Well-Test, on détermine si la variacine isolée selon la présente invention est inactivée en présence d'une enzyme protéolytique ou en présence de la catalase.

Pour cela, on ajoute 5 mg/ml d'enzyme à un concentrat du surnageant de culture illustré à l'exemple 2. On laisse agir l'enzyme à température d'incubation pendant 60 min avant de déposer l'échantillon dans le puits d'Agar Well-Test.

Parallèlement, on effectue un témoin préparé avec le même concentrat à pH 7 et sans addition d'enzyme. On l'incube à 37° C pendant 60 min, avant de le déposer dans le puits d'Agar Well-Test, de manière à comparer les halos d'inhibition en présence d'enzyme au halo d'inhibition du témoin. Le diamètre du halo d'inhibition du témoin est de 27 mm.

Le tableau I ci-après présente les résultats obtenus avec les enzymes testées à l'aide de la souche indicatrice, *Lactobacillus helveticus* (N 2). Dans ce tableau, comme dans le tableau II, l'enzyme est désignée par son type, le nom du fournisseur et le numéro d'article du fournisseur. Le chiffre 0 signifie qu'il n'y a plus de halo, autrement dit que le pouvoir bactériocide de la variacine a été compromis par l'incubation avec l'enzyme. Le chiffre 27 indique qu'il y a encore un halo de 27 mm correspondant à la pleine activité du pouvoir bactériocide de la variacine.

**Tableau I**

| Enzymes | Température d'incubation (° C) | Innactivation (mm) |
|---|---|---|
| Catalase (SIGMA C-10) | 30 | 27 |
| Pronase E (SIGMA P-8038) | 37 | 0 |
| Protéinase K (MERCK 1000 144) | 37 | 0 |
| Ficin (SIGMA F-3266) | 37 | 0 |

Le tableau II ci-après présente les résultats obtenus avec les enzymes testées à l'aide de la souche indicatrice, *Lactobacillus bulgaricus* (YL 5).

**Tableau II**

| Enzymes | Température d'incubation (° C) | Innactivation (mm) |
|---|---|---|
| Catalase (SIGMA C-10) | 30 | 27 |
| Pronase E (SIGMA P-8038) | 37 | 0 |
| Protéinase K (MERCK 1000 144) | 37 | 0 |
| Ficin (SIGMA F-3266) | 37 | 0 |

Les résultats présentés dans les tableaux I et II montrent que toutes les enzymes protéolytiques suppriment le pouvoir bactériocide de la variacine. Ces résultats mettent en évidence le fait que la variacine est de nature protéique et que cette partie protéique est impliquée dans le pouvoir bactériocide.

Le fait que l'on n'observe aucune influence de la catalase sur le pouvoir bactériocide de la variacine, démontre aussi que l'inhibition de la croissance des deux souches indicatrices n'est pas due à l'activité antibactérienne de H₂O₂ qui est connu pour avoir une activité semblable à celle des bactériocines, puisque H₂O₂ aurait été dégradé par la catalase.

### Spectre d'inhibition du surnageant de culture contenant la variacine

A l'aide de l'Agar Well-Test, on détermine si le pouvoir bactériocide du surnageant de culture contenant la variacine selon la présente invention présente une activité inhibitrice de la croissance des différentes souches de spores et de bactéries. On détermine ainsi le spectre d'inhibition du surnageant.

Pour réaliser ces essais, on verse, dans des boîtes de Pétri, 15 ml d'un milieu classique inoculé avec 15 µl d'une culture de la souche à tester préparée durant la nuit précédente, de manière à obtenir une concentration bactérienne de 10⁵-10⁶ par ml de milieu standard. Le milieu classique est le milieu favorable à la croissance de la souche à tester.

Par ailleurs, lorsque la souche à tester doit croître à partir de spores, on inocule avec 10⁵-10⁶ de spores par ml de milieu de recouvrement.

On perce un trou de 5 mm de diamètre par boîte de Pétri. On y dépose un échantillon de 50 µl de surnageant de culture, illustré à l'exemple 1. On pré-incube à 4° C pendant 2 h puis on incube à une température favorable à la croissance de la souche testée durant le temps nécessaire pour qu'elle recouvre la plaque d'un gazon bactérien visible.

On caractérise l'effet ou le degré d'inhibition par le diamètre du halo d'inhibition observé. On considère que l'inhibition est très forte (++++) si le halo présente un diamètre de 18-28 mm, forte (+++) pour un diamètre de 10-17 mm, moyenne (++) pour un diamètre de 5-9 mm, faible (+) pour un diamètre de 1-4 mm et nulle (-) si l'on n'observe aucun halo.

On teste ainsi 32 souches de bactéries lactiques de différentes espèces et sous-espèces et l'on constate qu'aucune n'est résistante au surnageant. Le détail du résultat de ces tests est présenté dans le tableau III ci-après. Dans ce tableau III, comme dans les tableaux suivants, la désignation ou No de souche indiqué est le No qui lui est attribué dans la collection Nestlé (Adresse: NESTEC S.A., Centre de Recherche,Vers-chez-les-Blanc, CH-1000 Lausanne 26, Suisse). La température indiquée est la température d'incubation durant le test. Le milieu indiqué est le milieu standard favorable à la croissance de la souche à tester.

**Tableau III**

| Espèces | No | T(°C) | Milieux | Inhibition |
|---|---|---|---|---|
| *Lactococcus lactis* | SL2 | 37 | MRS | +++ |
| *Lactobacillus helveticus* | N 2 | 37 | MRS | +++ |
| | N 258 | 37 | MRS | +++ |
| | N 262 | 37 | MRS | +++ |
| | N 271 | 37 | MRS | +++ |
| | LBL 4 | 37 | MRS | ++++ |
| *Lactobacillus debrueckii subsp. lactis* | N 9 | 37 | MRS | ++ |
| | N 62 | 37 | MRS | ++ |
| *Lactobacillus delbrueckii subsp. bulgaricus* | LFi 1 | 37 | MRS | +++ |
| | LFi 5 | 37 | MRS | +++ |
| | YL 5 | 37 | MRS | +++ |
| | YL 18 | 37 | MRS | +++ |
| *Lactobacillus delbrueckii subsp.* | N 8 | 37 | MRS | +++ |
| *delbrueckii* | N 187 | 37 | MRS | +++ |
| *Lactobacillus acidophilus* | La 3 | 37 | MRS | +++ |
| | La 10 | 37 | MRS | ++ |
| | La 27 | 37 | MRS | ++ |
| | La 28 | 37 | MRS | ++ |
| *Lactobacillus johnsonii* | LA 1 | 30 | MRS | ++ |
| *Lactobacillus plantarum* | 60 | 30 | MRS | + |
| | 3.4 RP | 30 | MRS | + |
| *Lactobacillus sake* | LSK | 30 | MRS | + |
| *Lactobacillus curvatus* | 18 | 30 | MRS | ++ |
| *Leuconostoc carnosum* | LCA 3 | 37 | M 17 | ++++ |
| *Leuconostoc mesenteroides subsp. mesenteroides* | A 74 | 30 | MRS | + |
| | B 50 | 30 | MRS | + |
| *Streptococcus thermophilus* | SFi 13 | 37 | Elliber | ++ |
| | SFi 16 | 37 | Elliber | ++ |
| | SFi 21 | 37 | Elliber | ++ |
| | SFi 25 | 37 | Elliber | ++ |
| | ST 11 | 37 | Elliber | ++ |
| | ST 1 | 37 | Elliber | ++ |

Sur ce tableau III, on constate que le spectre d'inhibition du surnageant est large au sens que pour les différentes espèces testées le degré d'inhibition est homogène.

On considère également que le spectre d'inhibition du surnageant d'une culture produisant la variacine de l'invention est large au sens qu'il ne se limite pas aux espèces de bactéries lactiques mais qu'il s'étend à d'autres espèces de bactéries Gram positives, notamment aux bactéries indésirables ou pathogènes *Listeria* *innocua, Listeria welhia, Listeria monocytogenes,* et aux spores des *Bacilli*, par exemple, comme en font foi les résultats présentés dans le tableau IV ci-après.

**Tableau IV**

| Espèces | No | T (° C) | Milieux | Inhibition |
|---|---|---|---|---|
| *Enterococcus faecalis subsp. faecalis* | J | 37 | Elliber | ++ |
| *Enterococcus faecium* | D 325 | 37 | Elliber | ++ |
| | MB 26 | 37 | Elliber | ++ |
| *Listeria innocua* | 7 | 30 | BHI | +++ |
| *Listeria monocytogenes* | FSM 122 | 30 | BHI | ++++ |
| *Listeria welhia* | 2 | 30 | BHI | ++ |
| *Bacillus subtilis* (spores et cellules végétatives) | A 1 | 30 | BHI | +++ |
| | A 2 | 30 | BHI | ++ |
| | A 3 | 30 | BHI | ++ |
| | A 4 | 30 | BHI | ++ |
| | A 13 | 30 | BHI | +++ |
| | A 15 | 30 | BHI | ++ |
| | 24 | 30 | BHI | ++ |
| | 152 | 30 | BHI | - |
| *Bacillus cereus* (spores et cellules végétatives) | C 1 | 30 | BHI | ++ |
| | C 2 | 30 | BHI | ++ |
| | C 5 | 30 | BHI | ++ |
| | C 6 | 30 | BHI | ++ |
| | 79 | 30 | BHI | ++++ |
| | C 15 | 30 | BHI | + |
| *Bacillus amyloliquefaciens* | 226 | 30 | BHI | ++++ |
| *Bacillus polymyxa* | 252 | 30 | BHI | ++++ |
| *Bacillus liqueniformis* | 64 | 30 | BHI | ++++ |
| *Bacillus stéarothermophilus* | 10 | 30 | BHI | - |
| *Bacillus circulans* | 215 | 30 | BHI | ++++ |
| *Bacillus pumulus* (spores et cellules végétatives) | B 1 | 30 | BHI | ++ |
| | B 2 | 30 | BHI | +++ |
| | 213 | 30 | BHI | ++++ |
| *Clostridium botulinum* (spores et cellules végétatives) | 100003 | 45 | DRMC | ++ |
| | 100006 | 45 | DRMC | ++ |
| | 100019 | 45 | DRMC | ++ |
| | 100023 | 45 | DRMC | ++ |
| *Clostridium butyricum* (spores et cellules végétatives) | 102001 | 45 | DRMC | ++ |
| *Clostridium tyrobutyricum* (spores et cellules végétatives) | 107002 | 45 | DRMC | + |
| *Clostridium perfringens* (spores) | 103001 | 45 | DRMC | + |
| *Clostridium sporogenes* (spores et cellules végétatives) | 104001 | 45 | DRMC | + |
| *Clostridium acetobutilycum* (spores et cellules végétatives) | 106001 | 45 | DRMC | + |
| *Clostridium thermosaccharolyticum* (spores et cellules végétatives) | 105001 | 45 | DRMC | + |
| *Staphylococcus aureus* | 3 | 30 | BHI | + |
| | 15 | 30 | BHI | + |
| | 44 | 30 | BHI | + |
| | 60 | 30 | BHI | ++ |
| *Staphylococcus xylosus* | 1 | 30 | BHI | + |
| *Staphylococcus simulans* | 1 | 30 | BHI | + |
| *Staphylococcus carnosus* | 1 | 30 | BHI | ++ |
| | 14 | 30 | BHI | ++ |
| *Staphylococcus saprophyticus* | 1 | 30 | BHI | + |
| | 15 | 30 | BHI | + |
| *Staphylococcus warneri* | 1 | 30 | BHI | + |
| *Staphylococcus cohnii* | 3 | 30 | BHI | + |

Les résultats illustrés dans ce tableau IV permettent entre autre de prévoir des utilisations intéressantes de ce surnageant ou de la variacine purifiée, comme additif dans la préparation de produits alimentaires en tant qu'agent actif contre des bactéries pathogènes, notamment dans des produits de viande contre les *Clostridia*, dans des fromages contre *Listeria monocytogenes*, dans des mousses dessert contre *Bacillus cereus* et *Listeria*, ou dans des pâtes fraîches ou des sauces pour pâtes fraîches contre les *Bacilli* dont les souches ci-dessus tirent précisément leur origine, par exemple.

Enfin, la variacine n'exerce aucun effet d'inhibition de la croissance de bactéries Gram négatives, comme on peut le constater au vu des résultats illustrés dans le tableau V ci-après.

**Tableau V**

| Espèces | No | T(° C) | Milieux | Inhibition |
|---|---|---|---|---|
| *E. coli* | 1 | 30 | BHI | - |
| *Enterobacter chloacae* | 72 | 30 | BHI | - |
| *Salmonella anatum* | XIV/20 | 30 | BHI | - |
| *Salmonela thyphimurium* | XIV/274 | 30 | BHI | - |
| *Pseudomonas fluorescens* | 3 | 30 | BHI | - |

### Spectre d'inhibition d'un concentrat du surnageant contenant la variacine

On procède de la manière décrite ci-dessus, à l'exception du fait que l'on détermine l'effet d'inhibition de la croissance de différentes souches de spores et de bactéries produit par un concentrat de surnageant obtenu comme décrit à l'exemple 2.

On teste les mêmes espèces et sous-espèces que précédemment. Les résultats de ces tests sont présentés dans les tableaux VI, VII et VIII ci-dessous. La désignation ou No de souche indiqué est le No qui lui est attribué dans la collection Nestlé (Adresse: NESTEC S.A., Centre de Recherche,Vers-chez-les-Blanc, CH-1000 Lausanne 26, Suisse). La température indiquée est la température d'incubation durant le test. Le milieu indiqué est le milieu standard favorable à la croissance de la souche à tester.

**Tableau VI**

| Espèces | No | T(° C) | Milieux | Inhibition |
|---|---|---|---|---|
| *Lactococcus lactis* | SL2 | 37 | MRS | +++ |
| *Lactobacillus helveticus* | N 2 | 37 | MRS | ++++ |
| | N 258 | 37 | MRS | ++++ |
| | N 262 | 37 | MRS | ++++ |
| | N 271 | 37 | MRS | ++++ |
| | LBL 4 | 37 | MRS | ++++ |
| *Lactobacillus delbrueckii subsp. bulgaricus* | LFi 1 | 37 | MRS | ++++ |
| | LFi 5 | 37 | MRS | ++++ |
| | YL 5 | 37 | MRS | ++++ |
| | YL 18 | 37 | MRS | ++++ |
| *Lactobacillus debrueckii subsp. lactis* | N 9 | 37 | MRS | +++ |
| | N 62 | 37 | MRS | +++ |
| *Lactobacillus delbrueckii subsp. delbrueckii* | N 8 | 37 | MRS | ++++ |
| | N 187 | 37 | MRS | ++++ |
| *Lactobacillus acidophilus* | La 3 | 37 | MRS | ++++ |
| | La 10 | 37 | MRS | ++++ |
| | La 27 | 37 | MRS | +++ |
| | La 28 | 37 | MRS | +++ |
| *Lactobacillus johnsonii* | LA 1 | 30 | MRS | +++ |
| *Lactobacillus plantarum* | 60 | 30 | MRS | ++ |
| | 3.4 RP | 30 | MRS | ++ |
| *Lactobacillus sake* | LSK | 30 | MRS | +++ |
| *Lactobacillus curvatus* | 18 | 30 | MRS | +++ |
| *Leuconostoc carnosus* | LCA 3 | 37 | M 17 | ++++ |
| *Leuconostoc mesenteroides subsp. mesenteroide* | A 74 | 30 | MRS | ++ |
| | B 50 | 30 | MRS | ++ |
| *Streptococcus thermophilus* | SFi 13 | 37 | Elliber | +++ |
| | SFi 16 | 37 | Elliber | +++ |
| | SFi 21 | 37 | Elliber | +++ |
| | SFi 25 | 37 | Elliber | +++ |
| | ST 1 | 37 | Elliber | +++ |
| | ST 11 | 37 | Elliber | +++ |

**Tableau VII**

| Espèces | No | T (° C) | Milieux | Inhibition |
|---|---|---|---|---|
| *Enterococcus faecalis subsp. faecalis* | J | 37 | Elliber | +++ |
| *Enterococcus faecium* | D 325 | 37 | Elliber | +++ |
| | MB 26 | 37 | Elliber | +++ |
| *Listeria inaocua* | 7 | 30 | BHI | ++++ |
| *Listeria monocytogenes* | FSM 122 | 30 | BHI | ++++ |
| *Listeria welhia* | 2 | 30 | BHI | ++++ |
| *Bacillus subtilis* (spores et cellules végétatives) | A 1 | 30 | BHI | +++ |
| | A 2 | 30 | BHI | ++ |
| | A 3 | 30 | BHI | ++ |
| | A 4 | 30 | BHI | ++ |
| | A 13 | 30 | BHI | +++ |
| | A 15 | 30 | BHI | ++ |
| | 24 | 30 | BHI | ++ |
| | 152 | 30 | BHI | - |
| *Bacillus cereus* (spores et cellules végétatives) | C 1 | 30 | BHI | ++ |
| | C 2 | 30 | BHI | ++ |
| | C 5 | 30 | BHI | ++ |
| | C 6 | 30 | BHI | ++ |
| | 79 | 30 | BHI | ++++ |
| | C 15 | 30 | BHI | + |
| *Bacillus amyloliquefaciens* | 226 | 30 | BHI | ++++ |
| *Bacillus polymyxa* | 252 | 30 | BHI | ++++ |
| *Bacillus liqueniformis* | 64 | 30 | BHI | ++++ |
| *Bacillus stéarothermophilus* | 10 | 30 | BHI | - |
| *Bacillus circulans* | 215 | 30 | BHI | ++++ |
| *Bacillus pumulus* (spores et cellules végétatives) | B 1 | 30 | BHI | ++ |
| | B 2 | 30 | BHI | +++ |
| | 213 | 30 | BHI | ++++ |
| *Clostridium butyricum* (spores et cellules végétatives) | 102001 | 45 | DRMC | +++ |
| *Clostridium perfringens* (spores) | 103001 | 45 | DRMC | ++ |
| *Clostridium tyrobutyricum* (spores et cellules végétatives | 107002 | 45 | DRMC | ++ |
| *Clostridium sporogenes* (spores et cellules végétatives) | 104001 | 45 | DRMC | ++ |
| *Clostridium acetobutilycum* (spores et cellules végétatives) | 106001 | 45 | DRMC | ++ |
| *Clostridium thermosaccharolyticum* (spores et cellules végétatives) | 105001 | 45 | DRMC | ++ |
| *Clostridia botulinum* (spores et cellules végétatives) | 100003 | 45 | DRMC | +++ |
| | 100006 | 45 | DRMC | +++ |
| | 100019 | 45 | DRMC | +++ |
| | 100023 | 45 | DRMC | +++ |
| *Staphylococcus aureus* | 3 | 30 | BHI | ++ |
| | 15 | 30 | BHI | ++ |
| | 44 | 30 | BHI | + |
| | 60 | 30 | BHI | +++ |
| *Staphylococcus xylosus* | 1 | 30 | BHI | + |
| *Staphylococcus simulans* | 1 | 30 | BHI | + |
| *Staphylococcus carnosus* | 1 | 30 | BHI | +++ |
| | 14 | 30 | BHI | +++ |
| *Staphylococcus saprophyticus* | 1 | 30 | BHI | ++ |
| | 15 | 30 | BHI | ++ |
| *Staphylococcus warneri* | 1 | 30 | BHI | ++ |
| *Staphylococcus cohnii* | 3 | 30 | BHI | ++ |

**Tableau VIII**

| Espèces | No | T(° C) | Milieux | Inhibition |
|---|---|---|---|---|
| *E. coli* | 1 | 30 | BHI | - |
| *Enterobacter chloacae* | 72 | 30 | BHI | - |
| *Salmonella anatum* | XIV/20 | 30 | BHI | - |
| *Salmonela thyphimirium* | XIV/274 | 30 | BHI | - |
| *Pseudomonas fluorescens* | 3 | 30 | BHI | - |

Les résultats illustrés dans les tableaux VI, VII et VIII mettent en évidence l'efficacité accrue du concentrat de surnageant comme inhibiteur de la croissance de nombreuses souches testées par rapport au surnageant. On observe des spectres d'inhibition pour les mêmes espèces et sous-espèces mais à un taux d'inhibition supérieur.

Cela permet de prévoir la préparation d'un concentrat de surnageant de variacine, comme décrit à l'exemple 2, et son utilisation pour la lutte contre des bactéries pathogènes dans la préparation de produits alimentaires et de produits cosmétiques, par exemple.

### Résistance au pH

A l'aide de l'Agar Well-Test, on détermine si la variacine isolée selon la présente invention est pH-dépendante.

A cet effet, on inocule l'agar avec une souche indicatrice, comme décrit précédemment dans le test "Agar Well-Test". On utilise comme souche indicatrice, *Lactobacilus helveticus* (N 2).

On ajuste le pH d'un extrait du concentrat du surnageant de culture décrit à l'exemple 2 à un pH de 2 à 10 avec du NaOH 2N et/ou du HCl 2N, on l'incube à 37° C pendant 60 min puis on réajuste le pH à 6-7 avant d'en déposer un échantillon dans le puits d'Agar Well-Test.

Parallèlement, on effectue un témoin avec le même concentrat de surnageant à pH 7 et on l'incube à 37° C pendant 60 min, avant de déposer l'échantillon témoin dans le puits d'Agar Well-Test, de manière à comparer les halos d'inhibition des tests au halo d'inhibition du témoin. Le diamètre du halo d'inhibition du témoin est de 27 mm.

Dans le tableau IX, comme dans les tableaux X et XI, le chiffre 27 indique qu'il y a encore un halo de 27 mm correspondant à la pleine activité du pouvoir bactériocide de la variacine.

**Tableau IX**

| pH | Halos d'inhibition |
|---|---|
| 2 | 27 |
| 4 | 27 |
| 6 | 27 |
| 8 | 27 |
| 10 | 27 |

Les résultats indiqués dans le tableau ci-dessus mettent en évidence le fait que le pouvoir bactériocide de la variacine n'est pas compromis. On peut donc conclure que le pouvoir bactériocide de la variacine n'est pas pH-dépendant.

### Résistance à la chaleur

A l'aide de l'Agar Well-Test, on détermine si la variacine isolée selon la présente invention est thermodépendante.

A cet effet, on inocule l'agar avec une souche indicatrice, comme décrit précédemment dans le test Agar Well-Test. On utilise comme souche indicatrice, *Lactobacillus helveticus* (N 2).

On incube à 100° C pendant 15 à 60 min un extrait de concentrat du surnageant de culture décrit à l'exemple 2 ajusté à pH 7 avant d'en déposer un échantillon dans le puits d'Agar Well-Test.

Parallèlement, on effectue un témoin obtenu avec le même concentrat de surnageant à pH 7 et incubé à 37° C pendant 60 min. On peut ainsi comparer les halos d'inhibition des tests de température au halo d'inhibition du témoin. Le diamètre du halo d'inhibition du témoin est de 27 mm.

**Tableau X**

| Température (° C) | Temps d'incubation (min) | Halos d'inhibition (mm) |
|---|---|---|
| 100 | 15 | 27 |
| 100 | 30 | 27 |
| 100 | 60 | 27 |

Ces résultats mettent en évidence le fait que la variacine n'est pas thermodépendante. En effet, le pouvoir bactériocide de la variacine n'est pas compromis même après une incubation à 100° C pendant 60 min.

La résistance à la chaleur de la variacine est une caractéristique biochimique d'une grande importance pour l'utilisation de la variacine dans la préparation de produits alimentaires et cosmétiques. En effet, on peut utiliser la variacine, notamment sous forme d'extrait brut ou purifié, dans la préparation d'aliments pasteurisés, de manière à lutter contre la croissance de spores, telles que les *Bacilli*, qui sont résistantes à la chaleur, par exemple.

### Résistance à la chaleur et au pH

De plus, on teste la stabilité de la variacine en combinant le pH et la chaleur.

A cet effet, on inocule l'agar avec une souche indicatrice, comme décrit précédemment dans le test "Agar Well-Test". On utilise comme souche indicatrice, *Lactobacilus helveticus* (N 2).

On ajuste l'extrait de concentrat de surnageant de culture décrit à l'exemple 2 à pH 4 ou 7 avec du HCl 2N et/ou du NaOH 2N, on l'incube à 115° C pendant 20 min puis on réajuste son pH à 6-7 avant d'en déposer un échantillon dans le puits d'Agar Well-Test.

Parallèlement, on effectue un témoin obtenu à pH 7 à 37° C pendant 20 min, avant de déposer l'échantillon témoin dans le puits d'Agar Well-Test, de manière à comparer les halos d'inhibition des tests au halo d'inhibition du témoin. Le diamètre du halo d'inhibition du témoin est de 27 mm.

**Tableau XI**

| pH | Temps d'incubation (min) | Température d'incubation (° C) | Halo d'inhibition (mm) |
|---|---|---|---|
| 4 | 20 | 115 | 27 |
| 7 | 20 | 115 | 27 |

Les résultats indiqués dans le tableau ci-dessus mettent en évidence le fait que le pouvoir bactériocide de la variacine n'est pas compromis à pH 4 ou 7, combiné à une température élevée.

### Purification de la variacine

On inocule 4 l de milieu BHI avec une culture de *Micrococcus varians*, productrice de la variacine selon la présente invention. On incube cette culture standard toute une nuit à 30° C, sous agitation en aérobiose, puis on la centrifuge à 5000 g, de manière à recueillir le surnageant dans un récipient, dans lequel on ajoute 72 g de résine XAD-7 (Amberlite (R)).

On agite à 25° C pendant 30 min, pour faciliter l'adhésion des molécules de variacine sur la résine, puis le tout est transféré sur un verre frité où le surnageant est filtré sous vide.

On lave la résine successivement dans 3 tampons contenant du citrate de sodium 20 mM pH 4 et de l'isopropanol. Le premier tampon contient 10 % d'isopropanol, le second tampon contient 15 % d'isopropanol et le troisième tampon contient 20 % d'isopropanol.

On transfère la résine dans une colonne et l'on élue la variacine avec 700 ml de tampon contenant du citrate de sodium 20 mM pH 4 et 25 % d'isopropanol.
On contrôle le pouvoir bactériocide de la variacine à l'aide de l'Agar Well-Test comme décrit précédemment.

On mélange les fractions actives et on évapore l'isopropanol. On prépare une colonne S-Ressource pour FPLC (Pharmacia) de 5 ml en l'équilibrant avec du tampon de citrate de sodium 20 mM. On charge le mélange des fractions actives évaporé sur cette colonne, puis on élue le contenu de la colonne avec un tampon NaCl ayant un gradient de 100 mM à 400 mM.

On collectionne des fractions et on on vérifie le pouvoir bactériocide de la variacine ainsi purifiée à l'aide de l'Agar-Well-Test.

### Séquençage de la variacine

On séquence la partie N-terminale de la variacine purifiée des souches CNCM I-1586 et CNCM I-1587 en utilisant un séquenceur automatique 4774 d'Applied Biosystems.
On révèle ainsi la séquence peptidique de 5 acides aminés dont la séquence est identique à celle, pour la partie N-terminale de la séquence SEQ ID NO:1, décrite dans la liste des séquences ci-après.
Il n'a pas été possible de révéler la présence d'un peptide de plus de 5 acides aminés lors du séquençage.

De plus, on effectue une hydrolyse de la variacine purifiée des souches CNCM I-1586 et CNCM I-1587 par du HCl 6N pendant 10 min. On obtient 3 peptides que l'on isole classiquement par HPLC. On n'a pu séquencer qu'un seul des trois peptides isolés, car les deux autres comportent certainement des modifications peptidiques.
La séquence dudit peptide ainsi isolé est identique à celle comprenant les acides aminés 19 à 22 de la séquence SEQ ID NO:1, décrite dans la liste des séquences ci-après.

Enfin, on soumet une fraction contenant la variacine purifiée des souches CNCM I-1586 et CNCM I-1587 à une spéctrométrie de masse et on révèle un poids moléculaire pour la variacine de 2659 Dalton.

### Homologie

Une homologie entre la séquence de la lacticin 481 de *Lactococcus lactis* et celle de la variacine selon la présente invention a été mise en évidence. Cette homologie concerne notamment la séquence de la partie N-terminale des deux bactériocines. En effet, les acides aminés 1 à 5 de la séquence N-terminale de la variacine sont identiques aux acides aminés 3 à 7 de la séquence SEQ ID NO:8 de la lacticin 481, décrite dans la liste des séquences ci-après. Néanmoins, il ne s'agit que d'homologie partielle et non d'identité.

De plus, on révèle, par spectrométrie de masse, un poids moléculaire de 2900 Dalton pour la lacticin 481 excrétée alors que le poids moléculaire de la variacine excrétée est de 2659 Dalton, comme nous l'avons vu précédemment.

Lorsque l'on inocule la souche de *Lactococcus lactis*, productrice de la lacticin 481, en présence d'un extrait de la variacine, comme cela a été précédemment décrit dans le test du spectre d'inhibition, on observe une inhibition de la croissance de ladite souche. La souche de *Lactococcus lactis*, productrice de la lacticin 481, est immune vis à vis de sa propre bactériocine, la lacticin 481 mais ne l'est pas vis à vis de la variacine, produite par l'une des deux souches de *Micrococcus varians*, selon la présente invention. Ces résultats obtenus lors du test du spectre d'inhibition, décrit précédemment, confirment le fait que l'on est en présence de deux bactériocines différentes.

Les données génétiques ci-dessus mettent en évidence, le fait que la lacticin 481 et la variacine présentent des homologies de séquence mais ne sont pas identiques. Les données biochimiques ainsi que les données microbiologiques confirment le fait que la lacticin 481 et la variacine sont deux bactériocines différentes.

### Séquençage du gène de la variacine

On fabrique d'une manière classique la séquence nucléotidique dégénérée SEQ ID NO: 4 décrite dans la liste des séquences ci-après, qui correspond à la partie C-terminale du peptide de la variacine séquencé précédement. On rend ensuite radioactif le mélange de séquences SEQ ID NO: 4, par l'action de la T4 polynucléotide kinase.

On réalise d'une manière classique une préparation d'ADN chromosomique des souches CNCM 1-1586 et CNCM I-1587. On effectue une digestion de ladite préparation d'ADN par *Sal*I, *Sac*I, *Sph*I et *BamH*I, en suivant les recommandations des fournisseurs d'enzymes. On fait ensuite migrer 2 µg du produit de digestion sur un gel d'agarose. On lave l'ADN du gel avec du HCl 250 mM puis on transfert le produit de migration en milieu alcalin du gel sur une membrane "Zetaprobe" (Biorad). La membrane Zetaprobe est alors préhybridée à une température de 55° C, abaissée de 5° C toutes les 2 h jusqu'à 40° C, dans un milieu comprenant du SSC 6X, 1 % de SDS, et 0,25 % de lait écrémé. On hybride cette membrane à la sonde dégénérée radioactive présentant la séquence SEQ ID NO: 4 dans le milieu d'hybridation précédent et dans les mêmes conditions de température. On laisse alors incuber à 40° C pendant 4 h, puis on lave la membrane à 40° C dans du SSC 6X. Enfin on l'expose à - 80° C pendant 16 h sur un film d'autoradiographie.

L'hybridation permet de mettre en évidence différentes bandes de migration: une bande de 7 kb *Sal*I, une bande de 1,4 kb *Sac*I, une bande de 1,8 kb *BamH*I, et une bande de plus 15 kb *Sph*I.

On digère alors 5 µg de DNA génomique de la souche CNCM I-1586 avec l'enzyme de réstriction *BamH*I et on sépare un fragment de 1,6-2 kb par chromatographie sur gel d'agarose suivie d'une élution de la partie du gel le contenant. On ligue le fragment d'ADN élué au vecteur pK 19 (Gene, 56 (1987) 309-312) préalablement digéré par *BamH*I puis traité à la phosphatase intestinale de veau ( Boehringer Mannheim, part no 713023).

On transforme alors classiquement par le milieu de ligation la souche *Escherichia coli* BZ 234 (collection du Biozentrum - Université de Bâle, Suisse) rendue préalablement compétente. Les clones contenant l'insert sont identifiés sur milieu agar complémenté avec 50 µg/ml de kanamycin, 60 ng/ml d'IPTG (Boehringer Mannheim, part no 724815), 300 ng/ml de X-gal (Boehringer Mannheim, part no 651745) et incubés à 37° C durant 16 h.

Les colonies blanches contenant normalement un insert sont repiquées dans des microplaques constituées de 96 puits. On repique chaque colonie blanche dans un desdits puits contenant 150 µl de milieu LB supplémenté avec 50 µg/ml de kanamycin et incubées à 37° C pendant 20 h pour produire des mini-cultures.

On prépare deux amorces d'orientation opposée, du fait que l'on ne connaît pas l'orientation du gène dans le vecteur pK 19. A cet effet, on construit lesdites amorces en réalisant un montage à partir d'un fragment nucléotidique présentant la séquence SEQ ID NO:5 codant partiellement pour la lacticin 481 que l'on lie à l'une ou l'autre des sondes universelles des vecteurs pUC présentant les séquences SEQ ID NO: 6 au SEQ ID NO: 7.

On mélange 1 µl de chaque puits avec 100 pmoles de l'une desdites amorces, 6 µl de dNTPs 2 mM, 2,5 µl de tampon-Taq ( P.H. Stehelin & Cie AG, part TP05b), on recouvre le tout avec une goutte de Dyna-wax ( Finnzymes Oy, 02201 Espoo, Finlande), et l'on chauffe à 98° C pendant 10 min, de manière à lyser les bactéries, puis on réalise la PCR.

On révèle alors sur un gel d'éléctrophorèse une bande de 800 pb pour les clones positifs.

On sélectionne ainsi les clones positifs, on en extrait l'ADN plasmidique et l'on séquence par la méthode des didéoxynucléotides le fragment d'ADN cloné dans le vecteur pK 19 à l'aide d'un kit de séquençage (Pharmacia Biotech, part no 27-1682-01) et d'amorces universelles, puis d'amorces spécifiques basées sur la séquence ainsi obtenue.

On obtient ainsi une séquence nucléotidique SEQ ID NO:2 décrite dans la liste des séquences ci-après. Ladite séquence nucléotidique code pour la séquence SEQ ID NO: 1, correspondant à la séquence en acides aminés de la variacine, avant maturation.

Les exemples ci-après sont présentés à titre d'illustration du procédé de production et des utilisations de la bactériocine selon la présente invention. Les pourcentages y sont donnés en poids sauf indication contraire.

### Exemple 1

On inocule un milieu de culture BHI au quel on ajoute une culture contenant des germes de la souche de *Micrococcus varians*. On incube toute une nuit à 30° C sous agitation et en aérobiose après quoi le milieu contient 10⁸ germes de la souche par ml. On centrifuge la culture standard ainsi obtenue. On recueille le surnageant standard.

### Exemple 2

On obtient le concentrat de surnageant à partir de 750 ml dudit surnageant obtenu, comme décrit à l'exemple 1, auquel on ajoute 15 g de résine XAD-7 (Amberlite (R)). On agite le mélange 60 min à 4° C et on filtre ensuite le mélange à travers un filtre Schleicher & Schuell (Allemagne) n°604. Puis on lave le filtre avec du citrate de sodium 1 %, de manière à éluer toutes les protéines non adsorbées. On isole la résine, on la transfère dans un récipiant contenant du citrate de sodium et l'on agite le tout pendant 2 min. On transfère la résine avec le citrate de sodium dans une colonne et on élue le citrate de sodium avec 50 % d'acitonitrine et 0,1 % de TFA. L'éluat est évaporé puis resuspendu dans du tampon phosphate 50 mM à pH 6,8.

### Exemple 3

On produit 10 litres d'une culture de la souche *Micrococcus varians* dans un milieu BHI toute une nuit à 30° C sous agitation et en aérobiose. On ajoute alors directement à la culture 200 g de la résine XAD-7 (Amberlite), et l'on agite le tout doucement pendant 1 h à 4°C. On filtre ensuite le mélange à travers un filtre Schleicher & Schuell (Allemagne) n°604, puis on lave la résine retenue sur le filtre par 10 litres d'une solution d'acide acétique 50mM pH 5,2, afin d'éliminer les bactéries. On ajoute alors à la résine 450 ml d'une solution comprenant de l'éthanol 100% et de l'acétate d'ammonium 20mM, on filtre le tout pour éliminer la résine, puis on lyophilise le filtrat jusqu'à obtenir une poudre comprenant la variacine selon l'invention, qui peut être utilisée dans l'industrie alimentaire.

On détermine alors le pouvoir bactériocide de cette poudre préalablement diluée dans de l'eau, par l'agar well test décrit précédemment. Cette poudre présente au moins 10⁵ ua/g de poudre.

Finalement, on ajoute à une mousse de viande, au cours de sa préparation de manière traditionnelle, 0,5 g/kg de la poudre ci-dessus. On obtient donc une mousse de viande comprenant 50 ua/g de bactériocines capables d'inhiber totalement le développement de bactéries pathogènes, notamment les *Clostridia*.

### Exemple 4

Cet exemple conçerne la préparation d'une crème hydratante pour soins de la peau contenant 0,05 g/kg de la poudre décrite dans l'exemple 3, soit donc 5 ua/g de variacines capables d'inhiber le développement de bactéries indésirables sur la peau.

Pour fabriquer cette émulsion, on mélange les composants de la phase lipidique A et on la chauffe à 75°C. On prépare la phase aqueuse B, et on la chauffe également à 75°C, puis on l'ajoute à la phase lipidique A en brassant lentement, et on refroidit ensuite sous brassage lent jusqu'à température ambiante, soit environ 25°C. A cette température, on ajoute lentement les constituants C dans l'ordre de la formule.

| Phase lipidique A | % |
|---|---|
| Peg-6-stéarate, glycérate et peg-20-céthyl éther (peg:polyéthylène glycol) | 15 |
| Huile de vaseline | 5 |
| Huile de germe de blé stabilisée avec 0,1% de phénylindanes (antioxydant) et 1% de phospholipides de soja (voir EP94109355.1) | 3 |
| Huiles d'amandes douces | 2 |
| Alcool cétylique | 1 |
| Isostéaryl-isostéarate | 2 |
| 2-Octyl-docécyl-mystirate | 1 |
| Cire de lanoline | 1 |

| Phase aqueuse B | % |
|---|---|
| Méthylisothiazoline | 0,1 |
| Eau déminéralisée | 59,6 |
| Protéine de placenta humain | 2 |

| Additifs C | % |
|---|---|
| Propylèneglycol et extrait de calendula | 2 |
| 50% de collagène soluble dans de l'eau déminéralisée | 5,8 |
| Parfum | 0,3 |
| 2,5% de poudre de bactériocines selon l'ex.3 dans de l'eau déminéralisée | 0,2 |

### Exemple 5

On ajoute à une eau dentrifrice 0,5 g/kg de la poudre de bactériocines décrite à l'exemple 3. Ce dentifrice est ainsi capable d'inhiber le développement de bactéries pathogènes de la cavité buccale, et notamment *Streptococcus sobrinus*.

### Exemple 6

On pulvérise une solution comprenant la poudre de bactériocine de l'exemple 3 diluée dans de l'eau à raison de 1%, sur un produit alimentaire destiné à être stérilisé pour prévenir une post-contamination lors de l'emballage.

## Revendications

1. Bactériocine de *Micrococcus varians* **caractérisée par le fait qu'**elle présente la séquence en acides aminés SEQ ID NO:1.

2. Fragment nucléotidique codant pour la bactériocine de *Micrococcus varians* selon la revendication 1.

3. Fragment selon la revendication 2, **caractérisé par le fait qu'**il comprend les nucléotides 88 à 228 de la séquence SEQ ID NO:2.

4. Fragment nucléotidique selon la revendication 2 ou 3, **caractérisé par le fait qu'**il comprend les nucléotides 154 à 228 de la séquence SEQ ID NO:2.

5. Fragment nucléotidique selon la revendication 2 ou 3, **caractérisé par le fait qu'**il comprend les nucléotides 88 à 153 de la séquence SEQ ID NO:2.

6. Peptide signal de *Micrococcus varians* codé par la séquence nucléotidique selon la revendication 5.

7. Souche de *Micrococcus varians* productrice de la bactériocine selon la revendication 1, notamment les souches CNCM I-1586 et CNCM I-1587.

8. Utilisation de la bactériocine selon la revendication 1 et/ou d'une souche de *Micrococcus varians* productrice de ladite bactériocine dans la préparation de produits alimentaires et de produits cosmétiques.

## Claims

1. Bacteriocin of *Micrococcus varians*, **characterized in that** it has the amino acid sequence SEQ ID NO:1.

2. Nucleotide fragment which codes for the bacteriocin of *Micrococcus varians* according to claim 1.

3. Fragment according to claim 2, **characterized in that** it comprises nucleotides 88 to 228 of sequence SEQ ID NO:2.

4. Nucleotide fragment according to claim 2 or 3, **characterized in that** it comprises nucleotides 154 to 228 of sequence SEQ ID NO:2.

5. Nucleotide fragment according to claim 2 or 3, **characterized in that** it comprises nucleotides 88 to 153 of sequence SEQ ID NO:2.

6. Signal peptide of *Micrococcus varians* coded by the nucleotide sequence according to claim 5.

7. Strain of *Micrococcus varians* which produces the bacteriocin according to claim 1, in particular strains CNCM I-1586 and CNCM I-1587.

8. Use of the bacteriocin according to claim 1 and/or of a strain of *Micrococcus varians* which produces the said bacteriocin in the preparation of food products and cosmetic products.

## Patentansprüche

1. Bakteriocin aus *Micrococcus varians*, **dadurch gekennzeichnet, daß** es die Aminosäuresequenz SEQ ID NO:1 aufweist.

2. Nukleotidfragment, das für das Bakteriocin aus *Micrococcus varians* nach Anspruch 1 kodiert.

3. Fragment nach Anspruch 2, **dadurch gekennzeichnet, daß** es die Nukleotide 88 bis 228 der Sequenz SEQ ID NO:2 aufweist.

4. Nukleotidfragment nach Anspruch 2 oder 3, **dadurch gekennzeichnet, daß** es die Nukleotide 154 bis 228 der Sequenz SEQ ID NO:2 aufweist.

5. Nukleotidfragment nach Anspruch 2 oder 3, **dadurch gekennzeichnet, daß** es die Nukleotide 88 bis 153 der Sequenz SEQ ID NO:2 aufweist.

6. Peptidsignal aus *Micrococcus varians,* das von der Nukleotidsequenz nach Anspruch 5 kodiert wird.

7. Stamm von *Micrococcus varians*, der das Bakteriocin nach Anspruch 1 produziert, insbesondere die Stämme CNCM I-1586 und CNCM I-1587.

8. Verwendung des Bakteriocins nach Anspruch 1 und/oder eines dieses Bakteriocin erzeugenden Stamms von *Micrococcus varians* bei der Herstellung von Nahrungsmittelprodukten oder kosmetischen Produkten.
